# EUROPEAN PATENT SPECIFICATION

(11) **EP 4 251 079 B1**
(45) Date of publication and mention of the grant of the patent: **27.11.2024**
(21) Application number: 21801899.2
(22) Date of filing: 28.10.2021
(51) Int. Cl.: A61B 18/18

(54) **ENDOSCOPIC ELECTROSURGICAL INSTRUMENT, ELECTROSURGICAL APPARATUS COMPRISING SUCH ELECTROSURGICAL INSTRUMENT, AND ENDOSCOPIC ELECTROSURGICAL KIT**
ENDOSKOPISCHES ELEKTROCHIRURGISCHES INSTRUMENT, ELEKTROCHIRURGISCHE VORRICHTUNG MIT SOLCH EINEM ELEKTROCHIRURGISCHEN INSTRUMENT UND ENDOSKOPISCHES ELEKTROCHIRURGISCHES KIT
INSTRUMENT ÉLECTROCHIRURGICAL ENDOSCOPIQUE, APPAREIL ÉLECTROCHIRURGICAL COMPRENANT UN TEL INSTRUMENT ÉLECTROCHIRURGICAL, ET KIT ÉLECTROCHIRURGICAL ENDOSCOPIQUE

(30) Priority: 26.11.2020 GB 202018631
(43) Date of publication of application: 04.10.2023
(73) Proprietor: Creo Medical Limited, Chepstow, Monmouthshire NP16 5UH (GB)
(72) Inventor: HANCOCK, Christopher Paul, Chepstow (GB)
(74) Representative: Mewburn Ellis LLP
(86) International application number: PCT/EP2021/079982
(87) International publication number: WO 2022/111938

(56) References cited:
- WO-A1-2020/025481
- US-A- 5 908 445
- US-A1- 2017 014 202
- US-A1- 2019 069 951
- US-A1- 2019 374 285

## Description

### TECHNICAL FIELD

The invention refers to an endoscopic electrosurgical instrument and to an electrosurgical apparatus comprising such electrosurgical instrument. The invention further relates to an endoscopic electrosurgical kit.

### BACKGROUND

Endoscopic procedures often use an endoscopic camera to image a target area to be examined or to be treated. The treatment itself is performed by a separate endoscopic instrument which is placed either alongside the endoscopic camera or the endoscopic camera is replaced by the endoscopic instrument. In the former case, the body cavity to be treated or may not be large enough to house both the endoscopic camera and the endoscopic instrument. In this case, the replacement of the endoscopic camera by the endoscopic instrument may be employed. However, this results in a treatment which cannot be simultaneously monitored by the endoscopic camera. Thus, the operator may blindly perform the treatment. This may negatively affect the result of the treatment.

US 2019/069951 A1 discloses an electrosurgical instrument for use in minimally invasive surgical techniques that provides a small scale localized microwave field capable of precisely ablating tissue, e.g. in the lungs, through suitable selection of geometry and material for a radiating distal tip thereof. US 2019/374285 A1 refers to an energy conveying structure for invasive electrosurgery that provides for combined delivery of (i) RF or microwave electromagnetic energy for tissue treatment (e.g. ablation, coagulation or cutting), and (ii) optical radiation within a common structure that may form an instrument cable of a surgical scoping device. WO 2020/025481 A1 discloses an electrosurgical instrument for delivering radiofrequency (RF) and/or microwave energy into biological tissue that is also configured to obtain images of a treatment site performing invasive electrosurgery. US 2017/014202 A1 refers to a catheter for visualizing ablated tissue that comprises a catheter body. US 5,908,445 A discloses an imaging element characterizes tissue morphology by analyzing perfusion patterns of a contrast media in tissue visualized by the imaging element.

### SUMMARY

The invention is defined by the independent claims. The dependent claims describe preferred embodiments of the invention.

An endoscopic electrosurgical instrument comprises a feed structure, a radiating element and an image capturing device. The feed structure is configured to convey electromagnetic energy from a proximal end of the electrosurgical instrument to a region at a distal end of the electrosurgical instrument. The radiating element is arranged in the region at the distal end. The radiating element is connected to the feed structure to receive the electromagnetic energy from the feed structure. The radiating element is configured for radiating the electromagnetic energy into a target area. The image capturing device is arranged in the region at the distal and configured to generate an image of a part of the target area.

An endoscopic electrosurgical kit comprises an image capturing apparatus and a radiating apparatus. The image capturing apparatus comprises an elongated body having a proximal end and a distal end as well as an image capturing device arranged in a region at the distal end and configured to generate an image of a part of the target area. The radiating apparatus comprises a tubular instrument sheath, a feed structure, and a radiating element. The feed structure is arranged in or on the instrument sheath and configured to convey electromagnetic energy from the proximal end of the instrument sheath to the region at the distal end of the instrument sheath. The radiating element is arranged in or on the instrument sheath in the region at the distal and, connected to the feed structure and configured for emitting electromagnetic radiation into the target area. The instrument sheath surrounds at least part of the body and is detachably or removably placed on the outer surface of the body.

An electrosurgical apparatus comprises the electoral surgical instruments as described above and a generator for generating electromagnetic energy. The generator is connected to the feed structure.

The endoscopic electrosurgical instrument and/or the endoscopic electrosurgical kit can simultaneously image parts of the target area while treating those same parts with the electromagnetic radiation emitted by the radiating element. The use of the image capturing device omits optical fibres conducting the light from the target area to an optical sensor arranged remote of the target area. Thus, an outer diameter of the electrosurgical instrument and/or the endoscopic electrosurgical kit can be reduced. This allows the endoscopic electrosurgical instrument and/or the endoscopic electrosurgical kit to be used in smaller body cavities compared to devices incorporating optical fibres.

The endoscopic electrosurgical instrument and/or the endoscopic electrosurgical kit described herein can be manufactured having an outer diameter that is smaller than commonly available imaging apparatus. For example, if the imaging apparatus constituted a bundle of optical fibres which direct light to the target area to be treated and guide light from the target area to an optical chip arranged remote from the target area to generate the image, the bundle of optical fibres has a significant diameter adding to the diameter of the endoscopic instrument. Thus, body cavities having a small diameter cannot be treated with the above described imaging apparatus combined with the endoscopic electrosurgical instrument. The provision of a bundle of optical fibres increasing the diameter of the imaging apparatus can be avoided with the endoscopic electrosurgical instrument and/or the endoscopic electrosurgical kit.

The electrosurgical kit provides the electrosurgical capabilities in and/or on the instrument sheath which can be pulled over the endoscopic imaging capturing apparatus. Thus, the radiating apparatus in the form of an instrument sheath does not significantly increase the diameter to the endoscopic image capturing device. What is more, the image capturing apparatus can be separated from the radiating apparatus such that either the image capturing apparatus or the radiating apparatus in form of the instrument sheath can be a disposable element.

The endoscopic electrosurgical instrument and/or the endoscopic electrosurgical kit can be configured as a device for treating and imaging a cavity arranged in a body of a human being or animal, such as a mammal. "Endoscopic" may be understood in that the electrosurgical instrument and/or the electrosurgical kit are configured to be used with a surgical scoping device. The electrosurgical instrument and/or the endoscopic electrosurgical kit may be inserted into a cavity of the body on their own or are guided to the treatment area using a scoping device. For example, the electrosurgical instrument and/or the endoscopic electrosurgical kit can be inserted into a catheter or other types of scoping devices, such as an endoscope, bronchoscope, cystoscope, nephroscope, arthoscope, colonoscope, or laparoscope. In addition, the electrosurgical instrument and/or the endoscopic electrosurgical kit may be used as an endoscope, bronchoscope, cystoscope, nephroscope, arthoscope, colonoscope, or laparoscope.

The electrosurgical instrument may include an elongated body which provides the endoscopic capabilities of the electrical surgical instrument. The feed structure, the radiating element, and/or the image capturing device may be arranged in and/or on the elongated body.

The elongated body, the feed structure and/or the radiating element may be flexible in order to permit the distal end of the electrosurgical instrument to flex as it moves through the body to the target area. The target area maybe a cavity (or zone or region) or part of a cavity (or zone or region) in the body which is to be treated and/or imaged. For example, the radiating element emits electromagnetic radiation for ablating, stimulating, cutting, and/or coagulating tissue in the target area. In an embodiment, the target area may be understood to be a region or zone outside the electrosurgical instrument but at or near the distal end of the instrument.

The electrosurgical instrument has an elongated structure which can be constituted by the elongated body. The distal end of the electrosurgical instrument is that end of the elongated structure of the electrosurgical instrument which is to be inserted into a patient. The proximal end of the electrosurgical instrument is preferably connected to the generator and/or a display device, and may in use remain outside the patient. The length of the electrosurgical instrument or elongated structure extends from the distal end to the proximal end.

The electrosurgical instrument may have a rod-like structure. The electrosurgical instrument is flexible perpendicular to its direction of extension (corresponding to a longitudinal direction) and rigid in its directions of extension.

The elongated body may have a tubular structure, i.e. the elongated body may have a central passageway (or bore or lumen) extending from the distal end to the proximal end. The elongated body may be constituted as a sheath for insulating or shielding (e.g. protecting) other components of the electrosurgical instrument from the outside, such as fluids.

The radiating element is arranged near or close to the distal end of the electrosurgical instrument. This means, the radiating element is arranged less than 5%, less than 3% or less than 1% of the overall length of the electrosurgical instrument away from the distal end. Thus, the radiating element is approximately arranged at the distal end.

The radiating element may be configured to emit radio frequency radiation and/or microwave radiation. The radio frequency radiation may be used for cutting tissue while the microwave radiation may be used to ablate, coagulate, and/or stimulate tissue at the target area. The radiating element may comprise one or more conductive elements. In particular, if the radiating element is configured to emit radio frequency radiation, the radiating element includes at least two conductive elements which are arranged close to each other but isolated from one another. An electromagnetic field generated between the two conductive elements by the radio frequency electromagnetic energy supplied to the radiating element can be used to cut tissue.

The radiating element may have a dipole structure or a monopole structure; the latter may be used for emitting microwave radiation. The radiating element, preferably all conductive elements, are arranged in and/or on the elongated body of the electrosurgical instrument.

The feed structure is connected to the radiating element for supplying electromagnetic energy from the proximal end to the radiating element. The feed structure may include any shape of conductive material in order to transmit electromagnetic energy. The feed structure may be connected to the generator at the proximal end.

The image capturing device may be configured to transform optical information of a part of the target area into electrical or digital information. For example, the image capturing device is configured to transform electromagnetic radiation, such as light, emitted from a part of the target area into an electrical or digital signal or a series of electrical or digital signals which can be processed to create a visible image of the part of the target area. The electrical or digital signal or the series of electrical or digital signals may be regarded as an electrical image or digital image of the target area. The generation of the electrical image or digital image is executed in the region at the distal end of the electrosurgical instrument. The processing of the electrical or digital signal or the series of electrical or digital signals may be executed by the display device which may also include a display for displaying the visible image of the captured part of the target area.

The image capturing device may be configured to create an optical image of the part of the target area at the region at the distal end. The image capturing device may be configured to convert the optical image of the part of the target area into an electric signal or a series of electric signals, for example digital signals, from which the image of the part of the target area can be reproduced by means of a processing apparatus, such as a computer or processor with a display screen or the display device. The conversion of the optical image created by the image capturing device into the electrical or digital image (corresponding to the electric signal or the series of electric signals) may be executed in the region at the distal end.

The image capturing device may be configured to produce an electric signal or a series of electric signals which can be transformed into a two-dimensional or three-dimensional representation of a part of the target area. This means, the image capturing device does not necessarily transmit optical information from the distal end to the proximal end but transforms the optical information into electrical information in the region at the distal end.

The image capturing device is arranged near or close to the distal end of the electrosurgical instrument. This means, the entire image capturing device is arranged less than 5%, less than 3% or less than 1% of the overall length of the electrosurgical instrument away from the distal end. Thus, the image capturing device is approximately arranged at the distal end.

The image capturing device may include one or more lens devices and/or one or more optical sensors. The lens device may be used to project incoming light beams originating from the part of the target area onto the optical sensor (for creating an optical image of the part of the target area on the optical sensor). The lens device may include one or more optical lenses and/or an (adjustable) aperture. However, it could be that the lens device may be a pinhole. The optical sensor may be configured to convert the incoming light into electrical signals. For example, the optical sensor may be a complementary metal-oxide-semiconductor (CMOS) sensor or a charge-coupled device (CCD). The CMOS sensor may have up to 40.000 pixels. The CMOS sensor may be a quadratic or square chip having a length as well as a width down to 0.65 mm each. The lens device and/or the optical sensor may be arranged in the region at the distal end.

The image capturing device may be a camera or any other means for converting optical information into electrical information indicative of a part of the target area. The image capturing device may be configured to image visible light, ultraviolet light and/or infrared light. The image capturing device may also be configured to identify the location of markers at the target area.

The image capturing device may have a main optical axis which is defined by the respective lens device and/or the pinhole. The main optical axis optionally is parallel to the direction of a longitudinal extension of the electrosurgical instrument. In this case, the image capturing device is configured to look forwards. Alternatively or additionally, the main optical axis may be inclined to the direction of the longitudinal extension of the electrosurgical instrument. For example, the image capturing device is configured to look sideward; the main optical axis may be inclined by 90° to the direction of extension, or any other angle between 0° and 90°. The lens device and/or the pinhole may be arranged on a side surface of the electrosurgical instrument or the body.

The electrical signals generated by the image capturing device may be fed to a display device which is connected to the proximal end of the electrosurgical instrument. This device may include a processor and/or a display. The display device may be a computer having a monitor or screen. The electrical signals generated by the image capturing device are processed by the processor and displayed by the display.

The electrosurgical instrument may also include an illuminator arranged in the region at the distal end. The illuminator can include an illumination device and/or a distal end of an illumination fibre or a distal end of a plurality of illumination fibres. The illuminator is provided for illuminating the target area or a part of the target area, in particular that part of the target area that is captured by the image capturing device.

The illumination device may include one or more light sources which may include one or more LEDs, for example, positioned in the region at the distal end. The illumination device, in particular the light source, may be powered by an illumination wire which is connected to the illumination device and extends to the proximal end. The illumination wire may be connected to a separate power source or may be powered by the display device. Alternatively or additionally, the illumination device may be connected to the feed structure. The energy supplied by the feed structure may be used to power the illumination device. For example, the electromagnetic energy fed to the radiating element may be an AC (alternating current) voltage which is superimposed with a DC (direct current) voltage for powering the illumination device. In another embodiment, the AC voltage for the radiating element may also power the illumination device.

The illuminator may include the distal end of an optical fibre(s) - the illumination fibre(s). The distal end of the illumination fibre(s) emits light that is fed into the illumination fibre(s) at the other end, for example at the proximal end. Thus, the illuminator forms a part of the illumination fibre(s). For example, the illuminator is a lens or lens structure arranged at or fixed to the distal end of the illumination fibre or to the plurality of illumination fibres. The proximal end of the illumination fibre may be connected to a light source, such as a lamp or an LED, which can be arranged in the display device. The illumination fibre(s) may protrude from the proximal end of the body.

The illumination fibre and/or the lens attached to the illumination fibre at the distal end may have a maximal outer diameter of 10 µm, 100 µm or 500 µm. Thus, the illumination fibre does not significantly increase the diameter of the electrosurgical instrument. This is in contrast to known endoscopic cameras which include a collection fibre for transmitting light collected at the distal end to an optical sensor arranged at the proximal end. The collection fibre has an outer diameter which is regularly 10 times larger than the diameter of the illumination fibre. Therefore, the omission of the collection fibre allows to significantly reduce the diameter of the electrosurgical instrument.

The outer diameter (or the maximal outer diameter) corresponds to the longest distance between any two points on the perimeter of the illumination fibre (or any other component to which the outer diameter refers to) in a cross sectional view.

In an embodiment, a plurality of illumination fibres are provided which may be arranged in a ring in a cross-sectional view. Each illumination fibre extends at least from the proximal end through the elongated body to the distal end. A plurality of rings of illumination fibres may be provided. The ring of illumination fibres may surround the optical sensor.

The image capturing apparatus of the electrosurgical kit may have to same capabilities as the image capturing device described above. However, the elongated body as described above belongs to the image capturing apparatus. The image capturing apparatus of the electrosurgical kit may be considered an endoscopic camera.

The radiating apparatus may include the feed structure and the radiating element as described above. In the electrosurgical kit, both the feed structure and the radiating element are arranged in and/or on the instrument sheath. The instrument sheath may be a flexible element having a tubular shape. The instrument sheath may be made from a plastic material, such as rubber or a polymer.

The instrument sheath may be pulled over the image capturing apparatus or the image capturing apparatus is inserted into the instrument sheath. The instrument sheath may be stuck to the image capturing apparatus by means of friction force (e.g. an interference fit). For example, the instrument sheath may have an inner diameter which is slightly smaller than the outer diameter of the elongated body of the image capturing apparatus such that, upon pulling the instrument sheath over the elongated body of the image capturing apparatus, the instrument sheath is expanded in the radial direction. It is also possible to fix (e.g. via an adhesive or mechanical fixing) the outer surface of the body to the inner surface of the instrument sheath in order to fix the instrument sheath to the body of the image capturing apparatus.

The instrument sheath includes the radiating element and the feed structure. Thus, by attaching the instrument sheath to the image capturing apparatus, an electrosurgical instrument having the above described capabilities can be assembled. The image capturing apparatus and the radiating apparatus are separate elements before the electrosurgical kit is assembled. It is possible to attach different types of cameras to the same instrument sheath or to apply different types of instrument sheath, for example including different types of radiating elements or feed structures, to the same image capturing device. This allows to easily customize the image capturing apparatus and/or the radiating apparatus to the individual situation.

By using the instrument sheath, an endoscopic camera or image capturing apparatus can be retrofitted with an electrosurgical device to treat tissue in the target area.

In an optional embodiment, the electrosurgical instrument or the image capturing apparatus further comprises at least one wire which is connected to the image capturing device and extends to the proximal end.

The at least one wire may be provided for powering the image capturing device. Furthermore, the at least one wire is provided for transmitting the electrical signals generated by the image capturing device to the proximal end of the at least one wire. The proximal end of the at least one wire may be connected to the display device for example by means of a connector.

For example, two wires are provided: one for powering the image capturing device and one for transmitting the electrical signals generated by the image capturing device to the proximal end. Furthermore, the wire or the wires connected to the image capturing device may be bundled together with the illumination wire.

The at least one wire and/or the illumination wire may extend within the central passage within the elongated body. The passage may extend from the proximal end to the distal end. It is possible that the image capturing device is connected to the display device by a conductor other than a wire, for example a coaxial cable.

Additionally or alternatively, the feed structure is connected to the image capturing apparatus for powering the image capturing apparatus and/or for transmitting the electrical or digital signal or the series of electrical or digital signals from the distal end to the proximal end. In this case, the display device may be connected to the feed structure at the proximal end. A first filter (e.g. low pass filter) may be provided with the display device to suppress the electromagnetic energy in the RF or microwave range being fed to the processor of the display device. In addition, the generator may include a second filter (e.g. a high pass filter) to suppress interference with the electrical or digital signal or the series of electrical or digital signals generated by the image capturing device.

In an optional embodiment, the radiating element includes a first electrode and/or a second electrode. Optionally, the radiating element includes the first electrode and the second electrode which is spaced apart (e.g. isolated) from the first electrode, the radiating element being exposed to the surrounding of the electrosurgical instrument.

The first electrode and the second electrode may be provided for the emission of radio frequency radiation. The first electrode and the second electrode may be electrically insulated from each other by an insulator arranged between the first electrode and the second electrode. For example, the first electrode and the second electrode are inserted or placed on the elongated body or the instrument sheath. The elongated body or the instrument sheath may be made from an insulating material, such as a plastic material.

The first electrode and/or the second electrode are preferably exposed to the surrounding of the electrosurgical instrument if they are used for emitting radio frequency radiation. This means, the first electrode and/or the second electrode can be placed in contact with the tissue of the target area.

The first electrode either alone or in conjunction with the second electrode may be used to emit microwave radiation. The first electrode and/or the second electrode may be attached to the feed structure.

It is possible that the first electrode and/or the second electrode are embedded within electrosurgical instrument, in particular the elongated body or the instrument sheath. This arrangement of the first electrode and/or the second electrode is preferably used if the electrodes are intended for emitting microwave radiation.

The first electrode and/or the second electrode may be made from a metal material and can protrude from an outer surface of the electrosurgical instrument, for example from the outer surface of the elongated body or the instrument sheath.

In an optional embodiment, the first electrode and/or the second electrode have a spiral shape, a ring shape, a semi-circular shape, an arcuate shape, or a hemispherical (or dome) shape.

As discussed, the first electrode and the second electrode can be arranged on the outer surface of the body. The first electrode and a second electrode having spiral shapes may be interleaved while they are spaced from each other in order to avoid short circuits. For example, when viewed along the longitudinal direction of the body, the arrangement of the electrodes may be: a part of the first electrode, a part of the second electrode, a part of the first electrode, a part of the second electrode et cetera.

In case of a ring shape, the first electrode and/or the second electrode extend around a circumference of the electrosurgical instrument. The first electrode and the second electrode may be spaced apart in the longitudinal direction of the electrosurgical instrument. The first electrode and the second electrode may include a plurality of rings which are alternatingly arranged along the longitudinal direction of the electrosurgical instrument.

If the shape of the first electrode and/or the second electrode is arcuate or semi-circular, the first electrode and the second electrode may located at the same position in the longitudinal direction while the ends of the arcuate or semi-circular curve of the first electrode and the second electrode are spaced apart from each other and face each other.

In case of a dome shape or hemispherical shape, the dome-shaped or hemispherical first electrode and the hemispherical second electrode are arranged on opposing sides of the electrosurgical instruments while being spaced apart from each other.

In an optional embodiment, the feed structure includes at least one feed wire connected to the radiating element and extending to the proximal end.

For example, one feed wire is connected to the first electrode while another feed wire is connected to the second electrode. Each feed wire may include a single elongated conductor with a small diameter. The feed wires may be intertwined.

The provision of the feed wire allows to provide a feed structure having a small diameter. As such, a thin feed wire does not significantly increase the outer diameter of the electrosurgical instrument. Optionally, the feed wire is provided for transmitting only radiofrequency electromagnetic energy.

At least a part of the feed structure is printed on the outer surface of the elongated body, wherein preferably the feed structure comprises a silver print material.

The feed structure may be printed on the instrument sheath or on both the instrument sheath and the outer surface of the elongated body. The implementation of the feed structure as a print (or printed material), preferably a silver print, provides a feed structure requiring little space. The print may have a thickness in the micrometre to millimetre range. The print of the feed structure provides a feed of electrical energy without a substantial increase in the diameter of the elongated body and/or the instrument sheath.

The print may be made from a metal material and extends to or onto the radiating element. For example, the feed structure may be printed on the outer surface of an existing endoscopic camera while the radiating element is attached to the distal end region of the endoscopic camera. This does not significantly increase the outer diameter of the endoscopic camera. Thus, the endoscopic camera can be retrofitted with electrosurgical treatment capabilities without significantly increasing the outer diameter of the endoscopic camera.

It is also possible that the radiating element is printed on the elongated body and/or the instrument sheath. For example, two conductors are printed onto the electrosurgical instrument whereby one conductor is connected to the first electrode and the other conductors connected to the second electrode. The first conductor may be spaced apart from the second conductor in a circumferential direction. The first conductor and/or the second conductor extend along the longitudinal direction of the elongated body.

In an optional embodiment, the feed structure is covered by an insulation sheath.

The print of the feed structure may be exposed to the surroundings of the electrosurgical instrument. The first conductor and/or a second conductor may thus apply electromagnetic energy to the tissue at any point along the extension from the proximal end to the distal end. Although the emission of electromagnetic energy can be minimised by spacing the two conductors far apart, this can be significantly reduced by the insulation sheath. Like the instrument sheath discussed above, the insulation sheath is a tubular member which can be pulled over the electrosurgical instrument, in particular over the elongated body. The insulation sheath, like the instrument sheath and/or the elongated body, may be made from a flexible electrically non-conductive material such as rubber, a polymer or other plastic materials. It is to be understood that in some embodiments both an instrument sheath and an insulation sheath may be provided, with the insulation sheath surrounding the instrument sheath.

The insulation sheath may be fixed to the elongated body by friction force. The insulation sheath may be pulled over the elongated body after the application of the printed feed structure.

In an optional embodiment, the feed structure includes a coaxial cable (or coaxial transmission line) comprising an inner conductor, an outer conductor coaxially arranged with the inner conductor and electrically insulated from the inner conductor by a dielectric material.

The coaxial cable may be provided if microwave radiation is supplied through the feed structure. The coaxial cable may have the usual configuration of an inner conductor and outer conductor separated by a dielectric material or a dielectric layer. The outer conductor may be covered (e.g. surrounded) by an electrically insulating cover (or jacket or tube), for example made from a plastic material, such as rubber or a polymer. Preferably, the coaxial cable is connected to the radiating element and extends to the proximal end.

The coaxial cable, the feed wire and/or the printed feed structure may be used alternatively or additionally to each other. For example, the coaxial cable may be used for transmitting microwave energy, while the feed wire and/or the printed feed structure may be employed for supplying radiofrequency energy.

In case of the presence of a coaxial cable, the elongated body may be constituted by the electrically insulating cover of the coaxial cable. For example, the electrically insulating cover extends (e.g. axially) beyond the outer conductor and the inner conductor at the distal end for housing the image capturing device. In an alternative embodiment, the image capturing device is attached to the distal end of the coaxial cable. However, it is also possible that the coaxial cable is arranged within the elongated body.

In an optional embodiment, the wire extends within the dielectric material, e.g. within a bore formed between the inner and outer conductors of the coaxial cable.

The wire may be completely embedded within the dielectric material. In this case, no additional space is needed for the wire. This helps to provide an electrosurgical instrument having a small outer diameter.

In an optional embodiment, the inner conductor is hollow and the wire extends in the hollow inner conductor, e.g. within a bore formed within the inner conductor.

Due to the Skin effect, the hollowness of the inner conductor does not significantly affect the transmission efficiency of the coaxial cable. The wire may by covered by an electrical insulator to prevent electrical connection between the wire and the inner and/or outer conductor. The wire having the insulator may be provided in the hollow inner conductor. The arrangement of the wire in the hollow inner conductor provides a compact structure of the electrosurgical instrument. In particular, the provision of the wire does not result in a (significant) increase of the diameter of the electrosurgical instrument. The hollow inner conductor provides a lumen extending from the proximal end to the end of the feed structure in the region at the distal end.

In an optional embodiment, the radiating element and/or the feed structure are arranged in and/or on the instrument sheath placed on the outer surface of the body, wherein preferably the instrument sheath is permanently fixed to the body.

The instrument sheath may be fixed to the body by means of an adhesive or mechanical means such as a clamp. The instrument sheath and the elongated body may form a unitary component.

The descriptions and preferred embodiment of the instrument sheath as described above in conjunction with the electrosurgical kit equally apply to the instrument sheath of the electrosurgical instrument. It is also possible that the instrument sheath is detachable or removable from the body as described above.

In an optional embodiment, the electrosurgical instrument further comprises an impedance transformer, preferably a microstrip impedance transformer, for matching the impedance of the feed structure to the impedance of a transmission line connected to the feed structure, wherein preferably the impedance transformer is connected to the feed structure at the proximal end.

The transmission line may be part of the generator. For example, the transmission line includes an internal feed line for supplying electromagnetic energy inside the generator and a connector (e.g. on an outer surface of a housing of the generator) to connect the feed line to the feed structure. It is also possible that the transmission line solely includes a connector for connecting the feed structure to a generator.

The feed structure may extend proximally beyond the proximal end of the elongated body for connecting the feed structure to the generator or the transmission line. Similarly, the wire may also extend proximally beyond the proximal end of the elongated body for connecting the wire to the display device.

The impedance transformer is any structure for matching the impedance of the transmission line, in particular the generator, to the impedance of the feed structure. The microstrip impedance transformer may be configured as described in WO 2018/146160 A1

The microstrip transformer may be employed with the coaxial cable having the hollow inner conductor.

The impedance transformer may be arranged at the proximal end of the feed structure. This means, when the feed structure is connected to the generator or the transmission line, the impedance transformer is directly adjacent to the connector.

In an optional embodiment, the outer diameter (e.g. the maximum outer diameter) of the electrosurgical instrument is less than 1.5 mm, preferably less than 1.2 mm.

The outer diameter of the electrical surgical instrument may be constituted by the outer surface of the elongated body or the outer surface of the instrument sheath. The elongated body and/or the instrument sheath do not necessarily need to have a circular cross-section. The cross section of the electrosurgical instrument or the electrosurgical kit may have an oval, rectangular or other shape. In this case, the outer diameter corresponds to the longest distance between any two opposite points on the perimeter of the cross-section of the elongated body or the instrument sheath.

This means that the coaxial cable may also have an outer maximal diameter of less than 1.5 mm, preferably less than 1.2 mm. The wire, the feed wire and/or the illumination wire may have a maximal outer diameter of less than 0.1 mm, 0.2 mm, 0.3 mm, 0.4 mm, 0.5 mm, 0.7 mm or 0.8 mm.

The defined outer diameters are achievable in that the image capturing device is provided such that no optical fibres are necessary which contributes to the reduction of the outer diameter. Furthermore, the use of the feed wires, the printed feed structure and/or the coaxial cable in which the wire is arranged helps to further reduce the outer diameter.

In an optional embodiment, the generator is configured to generate electromagnetic energy in the radio frequency range and/or the microwave frequency range. The microwave frequency range may be limited to microwave frequencies up to 915 MHz, for example 433 MHz.

In some embodiment, the small outer diameter of the electrosurgical instrument and, thus, of the coaxial feed cable may render the transmission of microwave energy having frequencies higher than 915 MHz inefficient because of high transmission losses. Thus, the generator may solely produce frequencies up to 915 MHz.

In case of the implementation of the feed structure as feed wires or a printed structure, the transmission of microwave frequency energy may result in too high transmission losses in conjunction with a significant heating the feed structure. Thus, generation of radiofrequency energy is may be preferred when implementing the feed structure as feed wires or a printed structure. The generator may solely generate electromagnetic energy in the above defined frequency ranges.

### BRIEF DESCRIPTION OF FIGURES

Embodiments of the invention will be discussed in conjunction with the accompanying drawings. Therein,
- Fig. 1: shows a schematic perspective view of an electrosurgical apparatus;
- Fig. 2: shows a cross-sectional view of an electrosurgical instrument of the electrosurgical apparatus according to Fig. 1;
- Fig. 3: shows a schematic perspective view of a region at the distal end of an electrosurgical apparatus according to a second embodiment;
- Fig. 4: shows a cross-sectional view of an electrosurgical instrument according to a third embodiment;
- Fig. 5: shows a schematic perspective view of the region at the distal end of an electrosurgical apparatus according to a fourth embodiment;
- Fig. 6: shows a cross-sectional view of the electrosurgical instrument of Fig. 5;
- Fig. 7: shows a schematic perspective view of a region at a distal end of an electrosurgical kit; and
- Fig. 8: shows a cross-sectional view of another embodiment of the electrosurgical kit.

### DESCRIPTION OF SOME EMBODIMENTS OF THE INVENTION

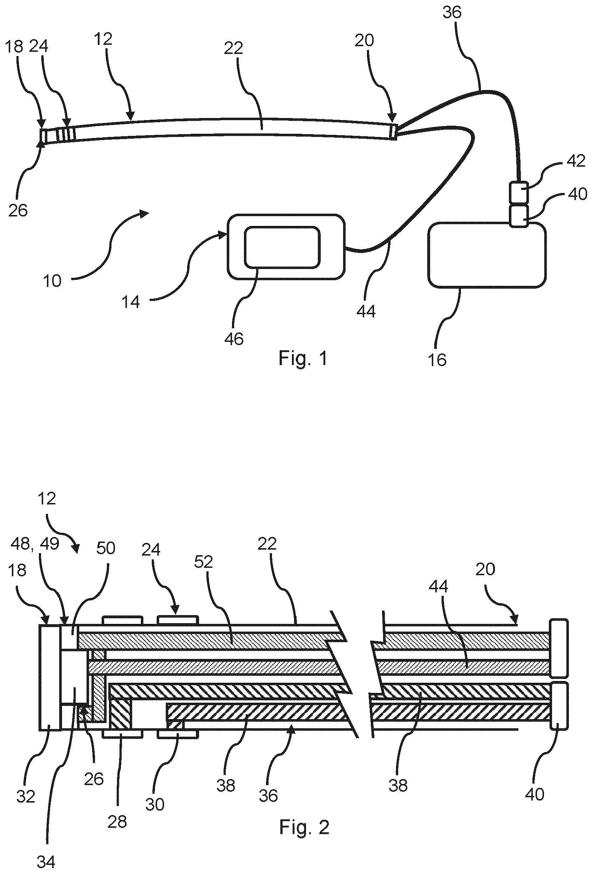

Fig. 1 shows a first embodiment of an electrosurgical apparatus 10 which comprises an endoscopic electrosurgical instrument 12, a display device 14, and a generator 16. The electrosurgical instrument 12 is configured to be inserted into a cavity of a body of a human or animal, such as a mammal.

The electrosurgical instrument 12 includes a distal end 18 and a proximal end 20. The distal end 18 of the electrosurgical instrument 12 is configured to be inserted into the cavity of the body. The length of the electrosurgical instrument 12 is preferably such that the proximal end 20 remains outside of the body.

The electrosurgical instrument 12 may include an elongated body 22 which extends from the distal end 18 to the proximal end 20. The extension of the body 22 or of the electrosurgical instrument 12 defines a longitudinal direction. The electrosurgical instrument 12 includes a radiating element 24 and an image capturing device 26 in a region at the distal end 18.

The elongated body 22 may have a tubular shape including a central passage in which various components are located as described in the following. The elongated body 22 can be made from a flexible material such as rubber or the like. The elongated body 22 and the electrosurgical instrument 12 are flexible perpendicular to the longitudinal direction such that they can be bent.

The radiating element 24 is arranged close or near the distal end 18 of the electrosurgical instrument 12 and, in particular, the body 22. The radiating element 24 is configured to emit electromagnetic radiation to a target area arranged within the cavity of the body. The electromagnetic radiation emitted by the radiating element 24 is provided for treating, in particular ablating, stimulating, cutting or coagulating, tissue at the target area. For example, the radiating element 24 is configured to emit radio frequency radiation or lower microwave radiation up to 915 MHz.

In the embodiment of Figs. 1 and 2, the radiating element 24 includes a ring-shaped first electrode 28 and a ring-shaped second electrode 30 which extend around the perimeter of the electrosurgical instrument 12, in particular the body 22. The first electrode 28 and a second electrode 30 are spaced apart in the longitudinal direction. The first electrode 28 and/or the second electrode 30 may protrude from an outer surface of the electrosurgical instrument 12, in particular the outer surface of the body 22. It is also possible that the first electrode 28 and/or the second electrode 30 are inserted into the electrosurgical instrument 12 such that they do not protrude from the outer surface of the electrosurgical instrument 12. The first electrode 28 and a second electrode 30 may be exposed to the surroundings of the electrosurgical instrument 12. The first electrode 28 and a second electrode 30 are made from a conductive material such as metal.

The image capturing device 26 is configured for converting optical signals into electrical signals. For example, the image capturing device 26 is capable of imaging a part of the target area and converting the information of the image into an electrical signal or a serious of the electrical signals. These signals may be processed by the imaging device 14 to display the image of the part of the target area.

The image capturing device 26 is arranged at the distal end 18 or in the region at the distal end 18. This means, all components of the image capturing device 26 are arranged at the distal end 18 or in the region at the distal end 18. For example, no component of the image capturing device 26 is arranged at the proximal end 20 or at the image capturing device 14.

The image capturing device 26 may be a camera and can include a lens device 32 and an optical sensor 34. The lens device 32 may include one or more lenses, an aperture and/or other devices suitable for imaging. The lens device 32 is preferably configured to project a part of the target area onto the optical sensor 34. The optical sensor 34 is configured to convert the impinging light into electrical signals. The optical sensor 34 may include a plurality of pixels. For example, the optical sensor 34 is a CMOS-sensor.

In the depicted embodiment, the main optical axis of the lens device 32 is parallel to the longitudinal direction of the electrosurgical instrument 12. Furthermore, the image capturing device 26 is arranged at the distal end 18. However, it is also possible that the main optical axis of the lens device 32 is inclined to the longitudinal direction. More generally, the image capturing device 26 may be configured to image a part of the target area which is arranged at the side of the electrosurgical instrument 12; the image capturing device 26 is configured to look in the radial direction. In this case, the image capturing device 26 may not to be arranged at the distal end 18 but can be arranged close or near the distal end 18.

The electrosurgical instrument 12 may include a feed structure 36 which, in the embodiment depicted in Figs. 1 and 2, includes two feed wires 38. The feed structure 36 is electrically connected to the radiating element 24. In detail, one feed wire 38 is connected to the first electrode 28 and the other feed wire 38 is connected to the second electrode 30.

The feed structure 36 is arranged in the passage of the body 22. The feed structure 36 may extend beyond the proximal end 20 of the elongated body 22. The proximal end of the feed structure 36 may include a connector 40 and/or an impedance transformer 42. The connector 40 may be employed for connecting the feed structure 36 to the generator 16.

The impedance transformer 42 may be provided to match the impedance of a transmission line to the impedance of the feed structure 36. The impedance transformer 42 may be a microstrip impedance transformer. The impedance transformer 42 may also be arranged within the generator 16. The impedance transformer 42 may be configured as described in WO 2018/146160 A1.

The transmission line may be any line for transmitting electromagnetic energy from the point of generation to feed structure 36 and can include the connector 40. For example, the transmission line may be a feed line arranged within the generator 16.

The connector 40 may have any form and/or shape which is suitable for connecting the feed structure 36 to the generator 16. For example, the connector 40 may be connected to both the feed wires 38.

The electrosurgical instrument 12 may include a wire 44 which is connected to the image capturing device 26 and to the display device 14. The wire 44 may be configured for powering the image capturing device 26 and/or for transmitting the electrical signals generated by the image capturing device 26 to the display device 14. Alternatively, a plurality of wires 44 may be provided. The wire 44 is arranged within the passage of the body 22. The wire 44 extends beyond the proximal end 20 of the elongated body 22. The wire 44 may be a thin metal wire.

The display device 14 may include a processor (not shown in the figures) and/or a display 46. The processor may be configured for processing the electrical signals generated by the image capturing device 26 and transmitted by the wire 44. The processor may be connected to the display 46 which is capable of displaying the image captured by the image capturing device 26.

Alternatively or additionally, the image capturing device 26 may be connected to the feed structure 36 (not depicted in the figures). The image capturing device 26 may be powered by the electromagnetic energy transmitted by the feed structure 26 to the region at the distal end 18. It is also possible that the feed structure 26 is used to transmit the electrical signals generated by the image capturing device 26 to the proximal end 20, for example to the display device 14. In this case, the display device 14 may be also connected to the feed structure 26.

The electrosurgical instrument 12 may further include an illuminator 48 which can comprise an illumination device 49 as depicted in Figs. 1 to 4. The illumination device 49 may be configured to illuminate at least a part of the target area, in particular the part of the target area which is imaged by the image capturing device 26. The illumination device 49 may include one or more light sources 50 which can be LEDs. In the embodiment shown in Figs. 1 and 2, the light sources 50 are arranged on the side of the optical sensor 34. The light sources 50 are arranged at the distal end 18. The light emitted by the light sources 50 may directed to the target area by means of the lens device 32. In other words, the lens device 32 is provided for both the illumination device 49 and the optical sensor 34. However, it is possible that the light sources 50 include lens structures for focusing or directing the emitted light onto the target area.

It is also possible that the light sources 50 are arranged on a side surface of the electrosurgical instrument 12, in particular, if the image capturing device 26 is configured to image the side of the electrosurgical instrument 12.

The electrosurgical instrument 12 may further include an illumination wire 52 which is connected to the illumination device 49 for powering the illumination device 49. The illumination wire 52 may run parallel to the wire 44 in the passage of the body 22. The illumination wire 52 may extend beyond the proximal end 20 of the body 22. The illumination wire 52 may also be connected to the display device 14. It is also possible that the wire 44 is additionally connected to the illumination device 49 for powering the illumination device 49. Alternatively or additionally, the feed structure 36 is connected to the illumination device 49 for powering the illumination device 49 (not depicted in the figures). In these cases, the illumination wire 52 may be omitted.

The generator 16 may be configured to generate radiofrequency energy and to feed this energy to the feed structure 36. The provision of the feed wires 38, the wire 44 and/or the illumination wire 52 allows to provide an outer diameter of the electrosurgical instrument 12 which is below 1.5 mm, preferably below 1.2 mm. Thus, the electrosurgical surgical instrument 12 can be advanced into cavities having a small diameter.

In use, the distal end 18 of the electrosurgical instrument 12 is inserted into the cavity of the body until the distal end 18 reaches the target area. The advance of the electrosurgical instrument 12 can be monitored by using the image capturing device 26. Similarly, the target area can be examined by using the image capturing device 26. This means, parts of the target area can be displayed on the display 46. If applicable, tissue at the target area may be treated by applying electromagnetic radiation emitted by the radiating element 24. For example, radiofrequency energy may be generated by the generator 16 and fed to the radiating element 24 by the feed structure 36. In particular, the first electrode 28 and the second electrode 30 form a dipole between which the radio frequency electrical field is generated which can be used for cutting tissue. The treatment process may be monitored by using the image capturing device 26.

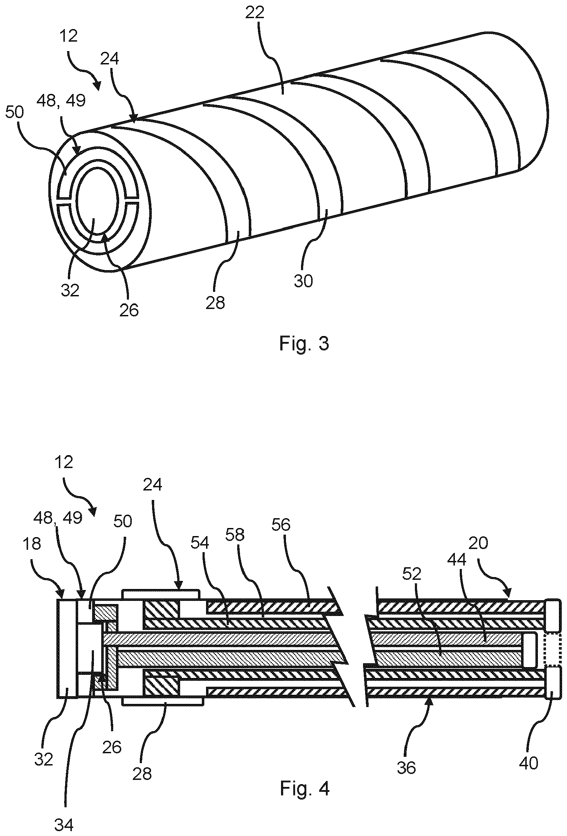

The electrosurgical instrument 12 depicted in Fig. 3 corresponds to the electrosurgical instrument 12 depicted in Figs. 1 and 2 except for the following differences:
The first electrode 28 and second electrode 30 have a spiral shape whereby the first electrode 28 and a second electrode 30 are intertwined. This means that a section of the first electrode 28 is arranged between two sections of the second electrode 30 when viewed along the longitudinal direction. Conversely, a section of the second electrode 30 is arranged between two sections of the first electrode 28 when viewed along the longitudinal direction.

The illumination device 49 includes two semi-circular light sources 50 or filaments which are arranged at the distal end 18. The semi-circular light sources 50 at least partially surround the lens device 32. Thus, in this embodiment, the light emitted by the light sources 50 does not directed through the lens device 32.

The electrosurgical instrument 12 depicted in Fig. 4 corresponds to the electrosurgical instrument 12 depicted in Figs. 1 and 2 except for the following differences:
The radiating element 24 is a monopole and not a dipole as with the embodiments depicted in Figs. 1 to 3. For example, the radiating element 24 solely includes a ring-shaped first electrode 28 which may be used for emitting low energy microwave radiation, for example microwave radiation with frequencies up to 915 MHz.

The feed structure 36 may include a coaxial cable which comprises an inner conductor 54, an outer conductor 56 and a dielectric material 58 electrically insulating the inner conductor 54 from the outer conductor 56. The inner conductor 54 is coaxially arranged to the outer conductor 56.

The elongated body 22 may be an electrically insulating cover covering the outer conductor 56 of the coaxial cable. In this embodiment, the shape of the electrosurgical instrument 12 is defined by the outer diameter of the coaxial cable. The inner conductor 54 may be connected to the first electrode 28.

In the embodiment depicted in Fig. 4, the inner conductor 54 is hollow. The wire 44 and/or the illumination wire 52 may be arranged inside the hollow inner conductor 54. The arrangement of the wire 44 and/or the illumination wire 52 within the hollow inner conductor 54 provides a compact structure resulting in a small outer diameter of the electrosurgical instrument 12, for example below 1.5 mm or 1.2 mm.

Alternatively, not depicted in the figures, the inner conductor 54 is not hollow and the dielectric material 58 is thicker in the radial direction compared to the embodiment depicted in Fig. 4. In this case, it is possible to arrange the wire 44 and/or the illumination wire 52 within the dielectric material 58. Thus, the wire 44 and/or the illumination wire 52 are embedded in the dielectric material 58. This also provides a compact structure resulting in a small outer diameter the electrosurgical instrument 12, for example below 1.5 mm or 1.2 mm.

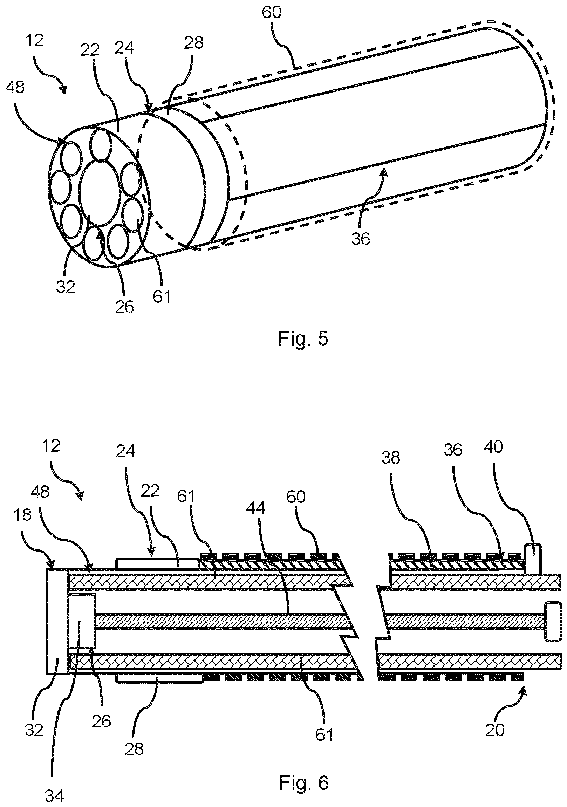

The electrosurgical instrument 12 depicted in Figs. 5 and 6 corresponds to the electrosurgical instrument 12 depicted in Figs. 1 and 2 except for the following differences:
The feed structure 36 is printed on the outer surface of the elongated body 22. The feed structure 36 may be a silver print. The thickness of the print in the radial direction may be in the micrometre or millimetre range. Thus, the provision of the feed structure 36 may not significantly increase the outer diameter of the electrosurgical instrument 12.

It is also possible that the radiating element 24 is printed on the outer surface of the elongated body 22. In the embodiment depicted in Figs. 5 and 6, the radiating structure 24 includes only the first electrode 28 which is a ring-shaped member. The radiating element 24 may be a narrow printed line. It is also possible, as depicted in Fig. 5, that the radiating element 24 extends over a larger range in the circumferential direction resulting in a broad printed line. This may help to reduce transmission losses. For example, the width of the radiating element 24 may be 10%, 20%, 30% or 50% of the circumference of the elongated body 22.

The printed radiating element 24 can be exposed to the surrounding of the electrosurgical instrument 12. This may result in that the radiating element 24 is in contact with tissue resulting in leakage of electromagnetic energy. Thus, an insulation sheath 60 may be provided which covers the outer surface of the elongated body 22 at least in that section in which the printed feed structure 36 is provided. The insulation sheath 60 may not cover the radiating element 24.

The insulation sheath 60 may be made from a flexible electrically insulating material such as rubber. The insulation sheath 60 may have a tubular shape. The insulation sheath 60 may be pulled over the electrosurgical instrument 12 after the application of the printed feed structure 36. The insulation sheath 60 may be fixed to the body 22 by friction. For example, an inner diameter of the insulation sheath 60 may be smaller than the outer diameter of the body 22 such that, upon pulling the insulation sheath 60 over the body 22, the insulation sheath 60 is radially expanded. However, it is possible that the insulation sheath 60 is additionally or alternatively fixed to the body 22 by adhesion.

The passage of the body 22 does not include the feed structure 36 such that the outer diameter of the elongated body 22 can be made smaller resulting in a reduced outer diameter of the electrosurgical instrument 12. In Fig. 6, the outer diameter of the electrosurgical instrument 12 is depicted to be the same as shown in Figs. 1 to 4 in order to highlight the space freed up by the presence of the printed feed structure 36. In practice, the outer diameter of the electrosurgical instrument 12 will be adapted to the diameter of the wire 44 and/or the illumination wire 52.

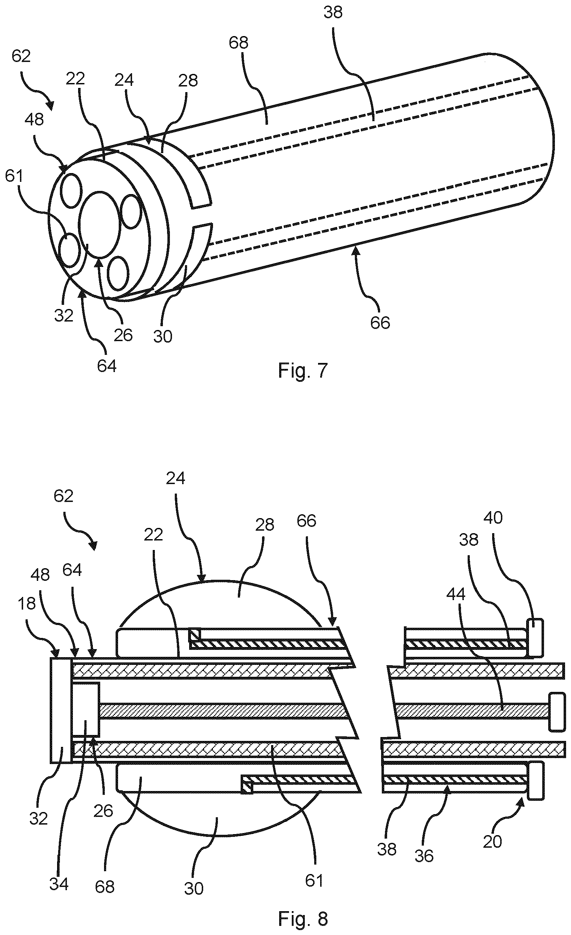

The illuminator 48 may be the end of an illumination fibre 61 or a bundle of illumination fibres 61 which can be constituted by optical fibres. The distal end of the illumination fibre 61 may include a lens or lens system for directing the light to the target area. Alternatively, as depicted in Figs. 5 to 8, the illumination fibres 61 are connected to the lens device 32. The illumination fibres 61 are arranged in a ring as visible in Fig. 5 and 7. The ring of illumination fibres 61 may surround the optical sensor 34. The distance between the illumination fibres 61 in the ring may be small (such that the illumination fibres contact each other) as depicted in Fig. 5 or the illumination fibres 61 are more loosely arranged in a ring around the optical sensor 34 as depicted in Fig. 7. The illumination fibres 61 may be uniformly or non-uniformly distributed around the optical sensor 34.

Each illumination fibre 61 may have an outer diameter of 10 µm, 100 µm or 500 µm. Each illumination fibre 61 may be connected to the display device 14 - more precisely to a light source, such as a lamp or LED, arranged within the display device. Each illumination fibre 61 guides the light generated by the light source arranged within the display device to the distal end 18 for illuminating the target area.

Alternatively, not depicted in Figs. 5 to 8, the illuminator 48 includes the illumination device 49 constituted by a plurality of light sources 50 which are arranged around the lens device 32. For example, four light sources 50 are equally distributed around the lens device 32. The light sources 50 may be arranged at positions corresponding to the illumination fibres 61 as discussed above.

Figs. 7 and 8 depict embodiments of an electrosurgical kit 62 which includes an image capturing apparatus 64 and a radiating apparatus 66. The image capturing apparatus 64 may be configured as a conventional endoscopic camera. For example, the image capturing apparatus 64 may include the components of the electrosurgical instrument 12 without the radiating element 24 and the feed structure 36. In particular, the image capturing apparatus 64 is the electrosurgical instrument 12 depicted in Fig. 6 without the radiating element 24 and the printed structure 36.

The radiating apparatus 66 may include the radiating element 24 and the feed structure 36 of any of the embodiments described above. The difference to the above described embodiments of the radiating element 24 and the feed structure 36 is the radiating element 24 and the feed structure 36 are fixed to an instrument sheath 68. This means the radiating apparatus 66 and the image capturing apparatus 64 are separate components. For example, the radiating apparatus 66 can be retrofitted to an existing image capturing apparatus 64 such as a conventional endoscopic camera. The instrument sheath 68 may be attached to the image capturing apparatus 64 similar to the fixation of the insulation sheath 60 the elongated body 22, for example by means of a friction fit.

The radiating apparatus 66 of Fig. 7 includes the first electrode 28 and the second electrode 30. Unlike the first electrode 28 and the second electrode 30 as depicted in Figs. 1 and 2, the first electrode 28 and the second electrode 30 have an arcuate, or more precisely a semi-circular, shape. The ends of the first electrode 28 face at the ends of the second electrode 30 while being spaced apart from each other. The first electrode 28 and a second electrode 30 are fixed to an outer surface of the instrument sheath 68. The first electrode 28 and the second electrode 30 protrude from the outer surface of the instrument sheath 68.

The radiating apparatus 66 of Figs. 7 and 8 includes two feed wires 38 which are respectively connected to the first electrode 28 and the second electrode 30. The feed wires 38 are embedded in the instrument sheath 68. The instrument sheath 68 may be made from a flexible electrically insulating material such as rubber. Thus, the feed wire 38 may be electrically insulated by being embedded within the instrument sheath 68.

However, it is also possible that the feed wires 38 of the radiating apparatus 66 are arranged on an inner surface of the instrument sheath 68. For example, the feed wires 38 are exposed at the inner surface of the instrument sheath 68. The feed wires 38 may protrude from the inner surface of the instrument sheath 68 or are flush with the inner surface of the instrument sheath 68, for example by partly embedding the feed wires 38 into the instrument sheath 68.

The feed structure 36 of the radiating apparatus 66 may also be printed on the instrument sheath 68, for example on the inner surface of the instrument sheath 68. It is possible that the feed structure 36 of the radiating apparatus 66 solely includes one feed wire 38 or one printed feed structure 36, for example if the radiating element 24 of the radiating apparatus 66 is a monopole.

The electrosurgical kit 62 of Fig. 8 is identical to the electrosurgical kit 62 of Fig.7 except for the following difference: the radiating element 24 of the embodiment depicted in Fig. 8 includes the first electrode 28 and the second electrode 30 in form of two hemispheres. The hemispheres are spaced apart from each other and are attached to the instrument sheath 68. A dome-shaped first electrode 28 and the second electrode 30 may be provided instead of the hemispheres.

The electrosurgical kit 62 can be used instead of the electrosurgical instrument 12. In particular, the outer diameter of the electrosurgical kit 62 and, thus, of the instrument sheath 68 may be below 1.5 mm or 1.2 mm. The electrosurgical kit 62 may be part of the electrosurgical apparatus 10 and can be used in conjunction with the display device 14 and/or the generator 16. The electrosurgical kit 62 may include the illuminator 48 having the features and characteristics as described in conjunction with the electrosurgical instrument 12.

In an alternative embodiment, the instrument sheath 68 may be permanently fixed to the elongated body 22. In this case, the resulting structure may be regarded as the electrosurgical instrument 12, in which the feed structure 36 and radiating element 24 are arranged in and/or on the instrument sheath 68 while the image capturing device 26, the wire 44 and/or the illumination wire 52are arranged in the elongated body 22.

It is also possible that certain components described or depicted in conjunction with one embodiment can be employed with another embodiment. For example, the illuminator 48 of one embodiment can be replaced by the illuminator48 of another embodiment. Similarly, the shape and form of the radiating element 24 of one embodiment can be replaced by the radiating element 24 of another embodiment. This also applies to the configuration of the feed structure 36.

## Claims

1. An endoscopic electrosurgical device, comprising:
an electrosurgical instrument (12),
a feed structure (36) configured to convey electromagnetic energy from a proximal end (20) of the electrosurgical instrument (12) to a region at a distal end (18) of the electrosurgical instrument (12),
a radiating element (24) arranged in the region at the distal end (18), the radiating element (24) being connected to the feed structure (36) to receive the electromagnetic energy therefrom, the radiating element (24) being configured to radiate the electromagnetic energy into a target area,
an image capturing device (26) arranged in the region at the distal end (18) and configured to generate an image of a part of the target area, and
an elongated body (22) extending from the proximal end (20) to the distal end (18),
**characterized in that** at least part of the feed structure (36) is printed on an outer surface of the body (22).

2. The electrosurgical device according to claim 1, further comprising at least one wire (44) connected to the image capturing device (26) and extending to the proximal end (20).

3. The electrosurgical device according to claim 1 or 2, wherein the radiating element (24) includes a first electrode (28) and/or a second electrode (30), wherein preferably the radiating element (24) includes the first electrode (28) and the second electrode (30) spaced apart from each other and exposed to a surrounding of the electrosurgical instrument (12), wherein, optionally, the first electrode (28) and/or the second electrode (30) have a spiral shape, a ring shape, arcuate, in particular a semi-circular, or a hemispherical shape.

4. The electrosurgical device according to any one of the preceding claims, wherein the feed structure (36) includes at least one feed wire (22) connected to the radiating element (24) and extending to the proximal end (20).

5. The electrosurgical device according to any one of the preceding claims, wherein the feed structure (36) comprises a silver printed material.

6. The electrosurgical device according to claim 5, further comprising an insulation sheath (60) surrounding at least part of the feed structure (36).

7. The electrosurgical device according to claim 5 or 6, wherein the radiating element (24) and/or the feed structure (36) are arranged in and/or on an instrument sheath (68) placed on the outer surface of the body (22), wherein preferably the instrument sheath (68) is fixed to the body (22).

8. The electrosurgical device according to any one of the preceding claims, wherein the feed structure (36) includes a coaxial cable comprising an inner conductor (54), an outer conductor (56) coaxially arranged with the inner conductor (54) and electrically insulated from the inner conductor (54) by a dielectric material (58).

9. The electrosurgical device according to claim 8, when dependent on claim 2, wherein:
the at least one wire (44) extends within the dielectric material (58) or
the inner conductor (54) is hollow and the at least one wire (44) extends in the hollow inner conductor (54).

10. The electrosurgical device according to any one of the preceding claims, further comprising an impedance transformer (42), preferably a microstrip impedance transformer, for matching an impedance of the feed structure (36) to an impedance of a transmission line connected to the feed structure (36), wherein preferably the impedance transformer (42) is connected to the feed structure (36) at the proximal end (20).

11. The electrosurgical device according to any one of the preceding claims, wherein an outer diameter of the electrosurgical instrument (12) is less than 1.5 mm, preferably less than 1.2 mm.

12. The electrosurgical device according to any one of the preceding claims, further comprising an illuminator (48) arranged in the region at the distal end (18) for illuminating the target area.

13. An endoscopic electrosurgical kit, comprising:
an image capturing apparatus comprising an elongated body (22) having a proximal end (20) and a distal end (18) as well as an image capturing device (26) arranged in a region at the distal end (18) and configured to generate an image of a part of a target area, and
a radiating apparatus comprising a tubular instrument sheath (68), a feed structure (36), and a radiating element (24), the feed structure (36) being arranged in and/or on the instrument sheath (68) and configured to convey electromagnetic energy from a proximal end (20) of the instrument sheath (68) to a region at a distal end (18) of the instrument sheath (68), the radiating element (24) being arranged in and/or on the instrument sheath (68) in the region at the distal end and connected to the feed structure (36) to receive the electromagnetic energy therefrom, the radiating element (24) being configured to radiate the electromagnetic energy into the target area, wherein the instrument sheath (68) surrounds at least a part of the elongated body (22) and is detachable from the elongated body (22), and
**characterized in that** the feed structure (36) is printed on the instrument sheath (68).

14. An electrosurgical apparatus, comprising:
the electrosurgical instrument (12) of any one of the claims 1 to 12 and
a generator (16) for generating electromagnetic energy, the generator (16) being connected to the feed structure (36).

15. The electrosurgical apparatus according to claim 14, wherein the generator (16) is configured to generate electromagnetic energy in a radio frequency range and/or at a microwave frequency up to 915 MHz.

## Patentansprüche

1. Endoskopische elektrochirurgische Vorrichtung, umfassend:
ein elektrochirurgisches Instrument (12), eine Speisestruktur (36), die dazu ausgelegt ist, elektromagnetische Energie von einem proximalen Ende (20) des elektrochirurgischen Instruments (12) zu einer Region an einem distalen Ende (18) des elektrochirurgischen Instruments (12) zu transportieren,
ein Abstrahlelement (24), das in der Region am distalen Ende (18) angeordnet ist, wobei das Abstrahlelement (24) mit der Speisestruktur (36) verbunden ist, um die elektromagnetische Energie von dieser zu empfangen, wobei das Abstrahlelement (24) dazu ausgelegt ist, die elektromagnetische Energie in einen Zielbereich abzustrahlen,
eine Bildaufnahmevorrichtung (26), die in der Region am distalen Ende (18) angeordnet und dazu ausgelegt ist, ein Bild eines Teils des Zielbereichs zu erzeugen, und
einen länglichen Körper (22), der sich vom proximalen Ende (20) zum distalen Ende (18) erstreckt,
**dadurch gekennzeichnet, dass** zumindest ein Teil der Speisestruktur (36) auf einer Außenfläche des Körpers (22) aufgedruckt ist.

2. Elektrochirurgische Vorrichtung nach Anspruch 1, ferner umfassend zumindest einen Draht (44), der mit der Bildaufnahmevorrichtung (26) verbunden ist und sich bis zum proximalen Ende (20) erstreckt.

3. Elektrochirurgische Vorrichtung nach Anspruch 1 oder 2, wobei das Abstrahlelement (24) eine erste Elektrode (28) und/oder eine zweite Elektrode (30) umfasst, wobei vorzugsweise das Abstrahlelement (24) die erste Elektrode (28) und die zweite Elektrode (30) voneinander beabstandet und gegenüber einer Umgebung des elektrochirurgischen Instruments (12) freiliegend umfasst, wobei gegebenenfalls die erste Elektrode (28) und/oder die zweite Elektrode (30) eine Spiralform, eine Ringform, eine bogenförmige, insbesondere eine halbkreisförmige oder eine halbkugelförmige Form aufweisen.

4. Elektrochirurgische Vorrichtung nach einem der vorangegangenen Ansprüche,
wobei die Speisestruktur (36) zumindest einen Speisedraht (22) umfasst, der mit dem Abstrahlelement (24) verbunden ist und sich bis zum proximalen Ende (20) erstreckt.

5. Elektrochirurgische Vorrichtung nach einem der vorangegangenen Ansprüche, wobei die Speisestruktur (36) ein silberbedrucktes Material umfasst.

6. Elektrochirurgische Vorrichtung nach Anspruch 5, ferner umfassend eine Isolierummantelung (60), die zumindest einen Teil der Speisestruktur (36) umgibt.

7. Elektrochirurgische Vorrichtung nach Anspruch 5 oder 6, wobei das Abstrahlelement (24) und/oder die Speisestruktur (36) in und/oder auf einer Instrumentenummantelung (68) angeordnet sind, die auf der Außenfläche des Körpers (22) platziert ist, wobei die Instrumentenummantelung (68) vorzugsweise auf dem Körper (22) fixiert ist.

8. Elektrochirurgische Vorrichtung nach einem der vorangegangenen Ansprüche,
wobei die Speisestruktur (36) ein Koaxialkabel umfasst, das einen Innenleiter (54) und einen Außenleiter (56), der koaxial mit dem Innenleiter (54) angeordnet und vom Innenleiter (54) durch ein dielektrisches Material (58) elektrisch isoliert ist, umfasst.

9. Elektrochirurgische Vorrichtung nach Anspruch 8 in Abhängigkeit von Anspruch 2, wobei:
der zumindest eine Draht (44) sich innerhalb des dielektrischen Materials (58) erstreckt, oder
der Innenleiter (54) hohl ist und der zumindest eine Draht (44) sich im hohlen Innenleiter (54) erstreckt.

10. Elektrochirurgische Vorrichtung nach einem der vorangegangenen Ansprüche,
ferner umfassend einen Impedanzwandler (42), vorzugsweise einen Mikrostreifen-Impedanzwandler, zum Anpassen einer Impedanz der Speisestruktur (36) an eine Impedanz einer mit der Speisestruktur (36) verbundenen Übertragungsleitung, wobei der Impedanzwandler (42) vorzugsweise am proximalen Ende (20) mit der Speisestruktur (36) verbunden ist.

11. Elektrochirurgische Vorrichtung nach einem der vorangegangenen Ansprüche, wobei ein Außendurchmesser des elektrochirurgischen Instruments (12) kleiner als 1,5 mm, vorzugsweise kleiner als 1,2 mm ist.

12. Elektrochirurgische Vorrichtung nach einem der vorangegangenen Ansprüche, ferner umfassend eine Beleuchtungsvorrichtung (48), die in der Region am distalen Ende (18) zum Beleuchten des Zielbereichs angeordnet ist.

13. Endoskopisches elektrochirurgisches Set, umfassend:
eine Bildaufnahmeeinheit, umfassend einen länglichen Körper (22) mit einem proximalen Ende (20) und einem distalen Ende (18), sowie eine Bildaufnahmevorrichtung (26), die in einer Region am distalen Ende (18) angeordnet und dazu ausgelegt ist, ein Bild eines Teils eines Zielbereichs zu erzeugen, und
eine Abstrahleinheit, umfassend eine schlauchförmige Instrumentenummantelung (68), eine Speisestruktur (36) und ein Abstrahlelement (24), wobei die Speisestruktur (36) in und/oder auf der Instrumentenummantelung (68) angeordnet und dazu ausgelegt ist, elektromagnetische Energie von einem proximalen Ende (20) der Instrumentenummantelung (68) zu einer Region an einem distalen Ende (18) der Instrumentenummantelung (68) zu transportieren, wobei das Abstrahlelement (24) in und/oder auf der der Instrumentenummantelung (68) in der Region am distalen Ende angeordnet und mit der Speisestruktur (36) verbunden ist, um die elektromagnetische Energie von dieser zu empfangen, wobei das Abstrahlelement (24) dazu ausgelegt ist, die elektromagnetische Energie in den Zielbereich abzustrahlen,
wobei die Instrumentenummantelung (68) zumindest einen Teil des länglichen Körpers (22) umgibt und vom länglichen Körper (22) lösbar ist, und **dadurch gekennzeichnet, dass** die Speisestruktur (36) auf der Instrumentenummantelung (68) aufgedruckt ist.

14. Elektrochirurgische Vorrichtung, umfassend:
ein elektrochirurgisches Instrument (12) nach einem der Ansprüche 1 bis 12, und
einen Generator (16) zum Erzeugen von elektromagnetischer Energie, wobei der Generator (16) mit der Speisestruktur (36) verbunden ist.

15. Elektrochirurgisches Gerät nach Anspruch 14, wobei der Generator (16) dazu ausgelegt ist, elektromagnetische Energie in einem Hochfrequenzbereich und/oder bei einer Mikrowellenfrequenz von bis zu 915 MHz zu erzeugen.

## Revendications

1. Dispositif électrochirurgical d'endoscopie, comprenant :
un instrument électrochirurgical (12),
une structure d'alimentation (36) configurée pour transporter de l'énergie électromagnétique à partir d'une extrémité proximale (20) de l'instrument électrochirurgical (12) vers une région au niveau d'une extrémité distale (18) de l'instrument électrochirurgical (12),
un élément rayonnant (24) agencé dans la région au niveau de l'extrémité distale (18), l'élément rayonnant (24) étant connecté à la structure d'alimentation (36) pour recevoir l'énergie électromagnétique à partir de celle-ci, l'élément rayonnant (24) étant configuré pour rayonner l'énergie électromagnétique jusque dans une zone cible,
un dispositif de capture d'image (26) agencé dans la région au niveau de l'extrémité distale (18) et configuré pour générer une image d'une partie de la zone cible, et
un corps allongé (22) s'étendant de l'extrémité proximale (20) à l'extrémité distale (18),
**caractérisé en ce qu'**au moins une partie de la structure d'alimentation (36) est imprimée sur une surface extérieure du corps (22).

2. Dispositif électrochirurgical selon la revendication 1, comprenant en outre au moins un fil (44) connecté au dispositif de capture d'image (26) et s'étendant vers l'extrémité proximale (20).

3. Dispositif électrochirurgical selon la revendication 1 ou 2, dans lequel l'élément rayonnant (24) comprend une première électrode (28) et/ou une seconde électrode (30), dans lequel, de préférence, l'élément rayonnant (24) comprend la première électrode (28) et la seconde électrode (30) espacées l'une de l'autre et exposées à un environnement de l'instrument électrochirurgical (12), dans lequel, facultativement, la première électrode (28) et/ou la seconde électrode (30) ont une forme de spirale, une forme d'anneau, une forme arquée, en particulier semi-circulaire, ou une forme hémisphérique.

4. Dispositif électrochirugical selon l'une quelconque des revendications précédentes, dans lequel la structure d'alimentation (36) comprend au moins un fil d'alimentation (22) connecté à l'élément rayonnant (24) et s'étendant vers l'extrémité proximale (20).

5. Dispositif électrochirurgical selon l'une quelconque des revendications précédentes, dans lequel la structure d'alimentation (36) comprend un matériau imprimé d'argent.

6. Dispositif électrochirurgical selon la revendication 5, comprenant en outre une gaine d'isolation (60) entourant au moins une partie de la structure d'alimentation (36).

7. Dispositif électrochirurgical selon la revendication 5 ou 6, dans lequel l'élément rayonnant (24) et/ou la structure d'alimentation (36) sont agencés dans et/ou sur une gaine d'instrument (68) placée sur la surface extérieure du corps (22), dans lequel de préférence la gaine d'instrument (68) est fixée au corps (22).

8. Dispositif électrochirugical selon l'une quelconque des revendications précédentes, dans lequel la structure d'alimentation (36) comprend un câble coaxial comprenant un conducteur interne (54), un conducteur externe (56) disposé coaxialement avec le conducteur interne (54) et isolé électriquement du conducteur interne (54) par un matériau diélectrique (58).

9. Dispositif électrochirurgical selon la revendication 8, lorsqu'elle dépend de la revendication 2, dans lequel :
le au moins un fil (44) s'étend à l'intérieur du matériau diélectrique (58) ou
le conducteur interne (54) est creux et le au moins un fil (44) s'étend dans le conducteur interne creux (54).

10. Dispositif électrochirurgical selon l'une quelconque des revendications précédentes, comprenant en outre un transformateur d'impédance (42), de préférence un transformateur d'impédance à microruban, pour faire correspondre une impédance de la structure d'alimentation (36) à une impédance d'une ligne de transmission connectée à la structure d'alimentation (36), dans lequel de préférence le transformateur d'impédance (42) est connecté à la structure d'alimentation (36) au niveau de l'extrémité proximale (20).

11. Dispositif élecrtochirugical selon l'une quelconque des revendications précédentes, dans lequel un diamètre extérieur de l'instrument électrochirurgical (12) est inférieur à 1,5 mm, de préférence inférieur à 1,2 mm.

12. Dispositif électrochirurgical selon l'une quelconque des revendications précédentes, comprenant en outre un dispositif d'éclairage (48) agencé dans la région au niveau de l'extrémité distale (18) pour éclairer la zone cible.

13. Kit électrochirurgical d'endoscopie, comprenant :
un appareil de capture d'image comprenant un corps allongé (22) présentant une extrémité proximale (20) et une extrémité distale (18), ainsi qu'un dispositif de capture d'image (26) disposé dans une région au niveau de l'extrémité distale (18) et permettant de générer une image d'une partie d'une zone cible, et
un appareil rayonnant comprenant une gaine d'instrument tubulaire (68), une structure d'alimentation (36) et un élément rayonnant (24), la structure d'alimentation (36) étant agencée dans et/ou sur la gaine d'instrument (68) et configurée pour transporter de l'énergie électromagnétique à partir d'une extrémité proximale (20) de la gaine d'instrument (68) vers une région au niveau d'une extrémité distale (18) de la gaine d'instrument (68), l'élément rayonnant (24) étant agencé dans et/ou sur la gaine d'instrument (68) dans la région au niveau de l'extrémité distale et connecté à la structure d'alimentation (36) pour recevoir l'énergie électromagnétique à partir de celle-ci, l'élément rayonnant (24) étant configuré pour rayonner l'énergie électromagnétique jusque dans la zone cible,
dans lequel la gaine d'instrument (68) entoure au moins une partie du corps allongé (22) et peut être détachée du corps allongé (22), et
**caractérisé en ce que** la structure d'alimentation (36) est imprimée sur la gaine d'instrument (68).

14. Appareil électrochirurgical, comprenant :
l'instrument électrochirurgical (12) selon l'une quelconque des revendications 1 à 12, et
un générateur (16) pour générer de l'énergie électromagnétique, le générateur (16) étant connecté à la structure d'alimentation (36).

15. Appareil électrochirurgical selon la revendication 14, dans lequel le générateur (16) est configuré pour générer de l'énergie électromagnétique dans une plage de fréquences radio et/ou à une hyperfréquence allant jusqu'à 915 MHz.
